# FASCICULE DE BREVET EUROPEEN

(11) **EP 0 467 759 B1**
(45) Date de publication et mention de la délivrance du brevet: **25.10.1995**
(21) Numéro de dépôt: 91401938.5
(22) Date de dépôt: 11.07.1991
(51) Int. Cl.: G01N 11/00, G01N 33/48

(54) **Dispositif de détection du changement de viscosité d'un électrolythe liquide par effet de dépolarisation**
Vorrichtung zum Feststellen der Viskositätsänderung eines flüssigen Elektrolyts mittels Depolarisierungswirkung
Device for detecting the change in viscosity of a liquid electrolyte using depolarisation effects

(30) Priorité: 20.07.1990 FR 9009285
(43) Date de publication de la demande: 22.01.1992
(73) Titulaire: DIAGNOSTICA STAGO (société anonyme), F-92602 Asnières (FR)
(72) Inventeur: Rousseau, Alain, F-94220 Charenton le Pont (FR)
(74) Mandataire: de Saint-Palais, Arnaud Marie

(56) Documents cités:
- EP-A- 0 164 473
- EP-A- 0 177 858
- EP-A- 0 325 874
- GB-A- 2 204 408
- GB-A- 2 211 614
- US-A- 3 502 559
- SOVIET INVENTIONS ILLUSTRATED, Section Electricité, semaine 8923, 19 juillet 1989, Derwent Publications Ltd., LONDON GB & SU-A-1420467
- LABORATORY PRACTICE vol. 24, no. 2, février 1975, GB page 79 E. CLAUSEN ET AL: "Use of the Clark oxygen electrode for viscosity measurements"
- CLINICAL CHEMISTRY. vol. 32, no. 3, mars 1986, WINSTON US pages 505 - 507; W.L. CHANDLER ET AL: "Evaluation of a new dynamic viscometer for measuring the viscosity of whole blood and plasma"

## Description

La présente invention concerne un dispositif de détection du changement de viscosité d'un fluide ayant les propriétés d'un électrolyte.

Plus particulièrement, il est destiné à la mesure du temps de coagulation d'une dose de sang mise en contact avec un réactif approprié.

Bon nombre de solutions ont été proposées pour mesurer la vitesse de coagulation du sang comme, par exemple, par introduction d'une bille ferromagnétique dans le fond d'un godet contenant le fluide à contrôler, et que l'on entraîne dans un mouvement périodique sous l'effet d'un champ magnétique extérieur et dans lequel on détecte les modifications des mouvements de cette bille, dues à la modification de l'état physique dudit fluide.

Par ailleurs, le document extrait de Clinical Chemistry de mars 1986, vol. 32, No. 3, pages 505 à 507 de W.L. Chandler et al. divulgue un viscosimètre pour mesurer la viscosité du sang, qui met en oeuvre une sonde vibrante introduite à une profondeur contrôlée dans l'échantillon à mesurer, et un circuit qui ajuste constamment la puissance des vibrations appliquées à la sonde de manière à ce que la sonde vibre de manière constante.

Ces dispositifs nécessitent l'utilisation d'un sang centrifugé ou plasma introduit en quantité notable dans un godet. En outre, les réactifs utilisés sont lyophilisés et doivent être reconstitués au moyen d'eau distillée au moment de leur usage.

D'autre part, le viscosimètre décrit dans le brevet SU-A-1420467 divulgue l'utilisation d'une cellule de Clark comprenant deux compartiments séparés par une membrane perméable aux gaz, le premier compartiment contenant deux électrodes immergées dans un électrolyte, le second compartiment étant destiné à recevoir un échantillon du liquide à analyser. Une telle cellule présente une structure complexe et ne permet pas d'obtenir une mesure indépendante du volume de l'échantillon.

Le but de la présente invention est de s'affranchir de ces inconvénients en proposant un dispositif à usage simplifié permettant, d'une part, une utilisation ambulatoire, à l'usage personnel de malades sous surveillance, devant effectuer des tests fréquents, et cela, sans mise en oeuvre de matériels compliqués, encombrants et nécessitant, après usage, une stérilisation, d'autre part, permettant la réalisation de tests intégrés pouvant contribuer à l'automatisation totale de la conduite des analyses : (sans reconstitution de réactif, sans problème de conservation, etc...).

A cet effet, l'invention repose sur l'effet de dépolarisation des électrodes dans une cellule électrochimique qui résulte de l'agitation de l'électrolyte.

Cet effet a été constaté par le Déposant, en relevant que la tension aux bornes d'une telle cellule lorsqu'elle débite, commence par suivre une loi d'évolution caractéristique du phénomène de polarisation pour marquer ensuite un palier qui signifie que l'agitation de l'électrolyte a eu un effet dépolarisant.

Le Déposant a ensuite constaté qu'en utilisant comme électrolyte, du sang ou du plasma mis en présence avec un réactif qui en provoque la coagulation au bout d'un certain temps, le palier était lui-même suivi d'une reprise de la loi normale d'évolution de la courbe de polarisation.

Des essais ont montré que le passage du palier à la reprise de la loi normale était critique et coïncidait avec l'instant de coagulation, tel qu'il a pu être déterminé par une méthode traditionnelle.

Le phénomène sera le même chaque fois qu'un électrolyte liquide subira une variation critique de sa viscosité qui aura pour effet de faire cesser son agitation.

D'autre part, l'une des difficultés des mesures chronométriques est de pouvoir détecter l'instant zéro quand le plasma ou le sang est mis en contact avec le réactif. Ce dispositif le donne d'emblée puisque dès que l'électrolyte est introduit, la pile alors constituée débite du courant qui indique très précisément le temps t = 0.

L'invention a donc pour objet un dispositif de détection du changement de viscosité d'un fluide ayant les propriétés d'un électrolyte se caractérisant en ce qu'il comporte : un réceptacle électriquement isolant muni de deux électrodes de nature métallique différente (exemple : cuivre et aluminium présentant un couple électrochimique exploitable), destinées à venir directement en contact avec ledit fluide ; des moyens d'engendrer un champ vibratoire à l'intérieur du réceptacle et des moyens de détecter les variations de tension entre les électrodes en fonction du temps, à partir de l'introduction du fluide dans le réceptacle, ces variations révélant une chute de tension qui se trouve artificiellement freinée jusqu'à un certain point à partir duquel la chute de tension reprend une course normale, et montrant que ledit changement s'est produit.

Un tel dispositif destiné à la mesure du temps de coagulation d'une dose de sang, mise en contact avec un réactif approprié, se caractérise également en ce que ledit réactif est déposé sous la forme d'une couche uniforme sur des surfaces baignées par l'électrolyte (constitué par exemple par du sang ou du plasma).

Selon une autre caractéristique de l'invention, un ensemble jetable destiné à être utilisé comme élément de l'appareil comporte un réceptacle en matière plastique et deux électrodes, agencé pour coopérer de manière amovible avec les moyens d'engendrer un champ vibratoire et les moyens de détecter la variation du potentiel des électrodes, débitant sur une résistance électrique déterminée.

Selon une autre caractéristique de l'invention, le dispositif comporte un support plan isolant sur lequel sont imprimés deux circuits électriques constituant les électrodes conductrices, celles-ci étant interdigitées pour former entre elles un espace isolant servant de réceptacle à une goutte de sang.

L'invention sera encore illustrée sans être aucunement limitée par la description qui va suivre faite en regard des dessins annexés sur lesquels :
La figure 1 est une vue schématique en coupe d'un dispositif de mesure de la vitesse de coagulation du sang selon l'invention ;
La figure 2 est une courbe illustrant le phénomène mis en oeuvre par l'invention ;
La figure 3 est une vue schématique d'une réalisation particulière des électrodes d'un dispositif selon l'invention.

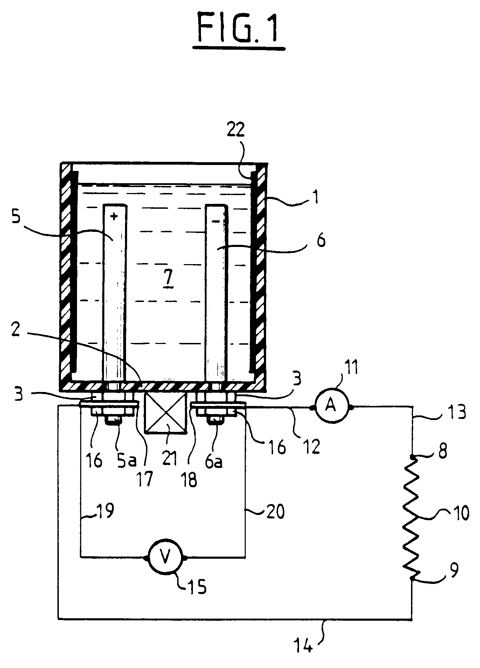

Le dispositif représenté sur la figure 1 est constitué essentiellement d'une cellule électrochimique tubulaire 1 fermée à sa partie inférieure par un fond 2. La cellule 1, réalisée en un matériau isolant, supporte une anode 5 et une cathode 6 fixées sur le fond 2, grâce à deux écrous 3 en prise avec des extrémités filetées 5a, 6a solidaires des électrodes 5, 6. Des moyens de traversée étanches (non représentés) du fond 2 sont prévus.

Cette cellule constitue un générateur d'électricité lorsqu'on introduit un électrolyte qui sera ici constitué par le sang dont on veut mesurer le temps de coagulation.

Les électrodes 5, 6 sont reliées électriquement aux bornes 8 et 9 d'une résistance consommatrice 10 par l'intermédiaire d'un ampèremètre 11 disposé en série avec la résistance 10 par un circuit de fils conducteurs 12, 13, 14. Les électrodes 5, 6 sont, par ailleurs, reliées à un voltmètre enregistreur 15 disposé en dérivation par les fils 19, 20 sur les extrémités 5a, 6a des électrodes 5, 6 constituant ainsi des bornes de raccordement sur lesquelles se vissent deux contre-écrous 16 enserrant deux cosses métalliques 17, 18 disposées aux extrémités respectivement des fils 14, 19 et des fils 12, 20.

Un réactif 22 destiné à provoquer la coagulation est déposé sur la paroi latérale de la cellule 1 avec une densité surfacique constante. De préférence, le réactif est mélangé à un gel que l'on fait ensuite sécher. Ceci facilite son étalement sur les supports concernés.

Ainsi réalisé, le dispositif constitue un ensemble jetable destiné à être utilisé comme élément de l'appareil.

Un générateur de vibrations 21 est couplé par exemple au fond 2 de la cellule 1.

Ces vibrations peuvent être engendrées par un générateur piézo-électrique de vibrations acoustiques ayant une fréquence de l'ordre de quelques centaines de Hertz ou bien elles peuvent être également engendrées par une table vibrante sur laquelle est posé le réceptacle 1.

L'appareil fonctionne de la manière suivante :

Lors de l'introduction du sang, la cellule commence à débiter dans la résistance 10 et simultanément, le générateur de vibrations 21 est mis en service pour provoquer l'agitation du sang.

Simultanément, on relève l'évolution de la tension aux bornes 5a, 6a de la cellule 1 pendant un temps allant de l'introduction du sang 7, fermant le circuit et initialisant le début de la mesure, jusqu'au moment où la chute de tension, artificiellement freinée, ne reprenne sa courbe normale de polarisation, temps correspondant à celui du changement d'état du sang, en fait à sa coagulation.

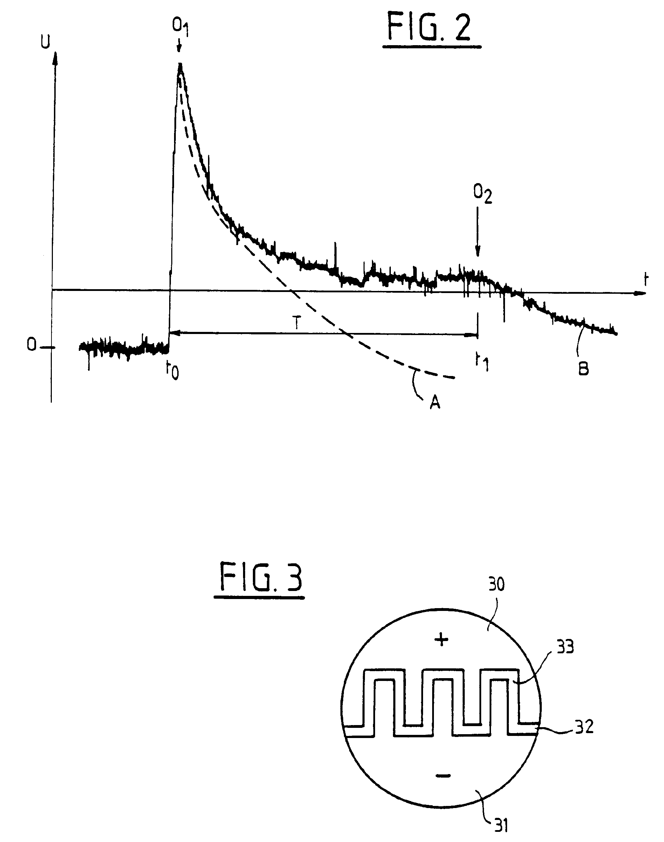

La courbe de la figure 2 illustre parfaitement le phénomène du freinage de la polarisation lorsque le sang 7 est soumis à vibrations.

La courbe fictive A montre une courbe connue de polarisation, alors que la courbe B montre la courbe obtenue concrètement lorsqu'intervient un phénomène vibratoire. Dans les deux cas, il s'agit de mesurer l'évolution d'une tension U en fonction d'un temps t.

Le début de polarisation correspond au point O₁ d'introduction du sang 7 dans la cellule 1 de l'appareil et cela à un temps t₀. La comparaison des courbes A et B montre nettement le freinage de la polarisation (courbe B) lorsque le sang est soumis à vibrations et cela jusqu'au point O₂ correspondant au point t₁ à partir duquel, le sang 7 étant entièrement coagulé, la courbe B de polarisation reprend son cours normal.

Le temps T, égal à l'écart entre t₀ et t₁, correspond au temps de coagulation du sang à contrôler.

L'opérateur peut relever directement ce temps sur l'enregistrement.

A titre de variation, il peut être mesuré par des dispositifs de traitement de signal associés à un moyen de comptage électronique, quine sera pas décrit ici.

Un avantage essentiel réside dans le fait que le temps mesuré est indépendant du volume de liquide.

L'exemple de réalisation représenté à la figure 3 répond au même principe ci-dessus décrit ; il ne diffère du précédent mode de réalisation qu'en ce qu'il est constitué d'électrodes positive et négative 30, 31 conductrices, obtenues par photo-impression sur un support plan isolant 32. Les circuits imprimés constituant les électrodes 30, 31 sont interdigités et forment entre eux un espace isolant 33. Une telle disposition permet non seulement une longueur de circuit développée importante dans une petite surface donnée mais permet également de s'affranchir du volume de sang à mettre en oeuvre, une simple goutte suffisant à couvrir au moins une partie des circuits ainsi formés. Comme précédemment, c'est le contact du sang entre les deux électrodes 30, 31 qui initialise la mesure.

Egalement, le réactif (non représenté dans cet exemple), est déposé au moins sur l'une des électrodes 30, 31, mais pour une meilleure homogénéité de la coagulation, il est déposé sur les deux.

Avantageusement, le circuit imprimé est obtenu sur le fond de la cellule 1 tel que décrit précédemment, le dispositif de raccordement et de mesure électrique étant inchangé.

## Revendications

1. Dispositif de détection du changement de viscosité d'un fluide ayant les propriétés d'un électrolyte,
caractérisé en ce qu'il comporte : un réceptacle électriquement isolant (1) muni de deux électrodes de nature métallique différente (5, 6), destinées à venir directement en contact avec ledit fluide (7) ; des moyens (21) d'engendrer un champ vibratoire à l'intérieur du réceptacle et des moyens (11 et/ou 15) de détecter les variations du courant entre les électrodes en fonction du temps, à partir de l'introduction du fluide dans le réceptacle (1), ces variations révélant une chute de tension qui se trouve artificiellement freinée jusqu'à un certain point (O2), à partir duquel la chute de tension reprend une course normale, et montrant que ledit changement s'est produit.

2. Dispositif selon la revendication 1,
caractérisé en ce que lesdits moyens de détection comportent un circuit fermé reliant les électrodes (5, 6) à une résistance (10) pour provoquer une polarisation de celles-ci et des moyens (11, 15) de mesurer la variation du courant entre les électrodes (5, 6), fonction du changement de viscosité.

3. Dispositif selon la revendication 1 destiné à la mesure du temps de coagulation d'une dose de sang (7) mise en contact avec un réactif approprié (22),
caractérisé en ce que ledit réactif (22) est déposé sous la forme d'une couche uniforme sur des surfaces baignées par l'électrolyte, ou de plusieurs couches superposées (polyréactif).

4. Dispositif selon les revendications 2 et 3,
caractérisé en ce que lesdits moyens de mesure (11, 15) de la variation de tension comportent des moyens de déterminer l'instant (t0) d'établissement de courant entre les électrodes lors de l'introduction du sang dans le réceptacle et l'instant (t1) où le courant subit une variation caractéristique de la coagulation.

5. Ensemble jetable destiné à être utilisé comme élément de l'appareil selon la revendication 3,
caractérisé en ce qu'il comporte un réceptacle en matière plastique (1) et deux électrodes (5, 6), agencé pour coopérer de manière amovible avec les moyens (21) d'engendrer un champ vibratoire et les moyens (11, 15) de détecter la variation de courant entre les électrodes (5, 6).

6. Appareil selon les revendications 2 et 3,
caractérisé en ce qu'il comprend un support plan isolant (32) sur lequel sont imprimés deux circuits électriques constituant les électrodes conductrices (30, 31), celles-ci étant interdigitées pour former entre elles un espace isolant servant de réceptacle à une goutte de sang.

7. Appareil selon la revendication 6,
caractérisé en ce qu'au moins l'une des faces des circuits imprimés constituant les électrodes (30, 31) constitue un support au réactif ou aux réactifs.

8. Appareil selon la revendication 1,
caractérisé en ce que lesdits moyens d'engendrer un champ vibratoire sont constitués par un générateur piézo-électrique de vibrations acoustiques ayant une fréquence de l'ordre de quelques centaines de Hertz.

9. Appareil selon la revendication 1,
caractérisé en ce que les moyens d'engendrer un champ vibratoire sont constitués par une table vibrante sur laquelle est posé le réceptacle.

## Patentansprüche

1. Vorrichtung zur Erfassung der Viskositätsänderung eines Fluids, das die Eigenschaften eines Elektrolyten hat,
dadurch gekennzeichnet, daß sie aufweist: einen elektrisch isolierenden Behälter (1) mit zwei Elektroden aus verschiedenen Metallen (5, 6), die direkt mit dem Fluid (7) in Kontakt kommen sollen, Mittel (21), um ein Vibrationsfeld innerhalb des Behälters zu erzeugen, und Mittel (11 und/oder 15), um die Stromänderungen zwischen den Elektroden in Abhängigkeit von der Zeit zu erfassen, ausgehend von der Einführung des Fluids in den Behälter (1), wobei diese Änderungen einen Spannungsabfall darstellen, der künstlich bis zu einem gewissen Punkt (O₂) gebremst wird, ausgehend von welchem der Spannungsabfall wieder einen normalen Weg aufnimmt und zeigt, daß die Änderung stattgefunden hat.

2. Vorrichtung nach Anspruch 1, dadurch gekennzeichnet, daß die Erfassungsmittel eine geschlossene Schaltung, die die Elektroden (5, 6) mit einem Widerstand (10) verbinden, um eine Polarisation der Elektroden zu erreichen, und Mittel (11, 15) aufweisen, um die Veränderung des Stroms zwischen den Elektroden (5, 6) zu messen, die von der Viskositätsänderung abhängt.

3. Vorrichtung nach Anspruch 1 zur Messung der Koagulationszeit einer Blutprobe (7), die mit einem geeigneten Reagens (22) in Kontakt gebracht wurde, dadurch gekennzeichnet, daß das Reagens (22) in Form einer gleichmäßigen Schicht auf vom Elektrolyten bedeckten Flächen oder in Form mehrerer übereinanderliegender Schichten (Polyreagens) aufgebracht wird.

4. Vorrichtung nach den Ansprüchen 2 und 3, dadurch gekennzeichnet, daß die Meßmittel (11, 15) der Spannungsveränderung Mittel zur Bestimmung des Zeitpunkts (t₀) der Erzeugung eines Stroms zwischen den Elektroden bei der Einführung des Bluts in den Behälter und des Zeitpunkts (t₁) enthalten, in dem der Strom eine für die Koagulation charakteristische Veränderung erfährt.

5. Wegwerfbare Einheit, die als Element des Geräts nach Anspruch 3 genutzt werden soll, dadurch gekennzeichnet, daß sie einen Behälter (1) aus Kunststoffmaterial und zwei Elektroden (5, 6) enthält und so ausgebildet ist, daß sie lösbar mit den Mitteln (21) zur Erzeugung eines Vibrationsfelds und den Mitteln (11, 15) zur Erfassung der Stromveränderung zwischen den Elektroden (5, 6) zusammenwirken kann.

6. Gerät nach den Ansprüchen 2 und 3, dadurch gekennzeichnet, daß es einen ebenen isolierenden Träger (32) aufweist, auf den zwei elektrische Schaltungen aufgeprägt sind, die die leitenden Elektroden (30, 31) bilden, wobei diese ineinandergeschoben sind, um zwischen sich einen isolierenden Raum zu bilden, der als Behälter für einen Bluttropfen dient.

7. Gerät nach Anspruch 6, dadurch gekennzeichnet, daß mindestens eine der Seiten der die Elektroden (30, 31) bildenden Druckschaltungen einen Träger für das Reagens oder die Reagentien bildet.

8. Gerät nach Anspruch 1, dadurch gekennzeichnet, daß die Mittel zur Erzeugung eines Vibrationsfelds aus einem piezoelektrischen Erzeuger von akustischen Vibrationen bestehen, die eine Frequenz der Größenordnung von mehreren hundert Hertz aufweisen.

9. Gerät nach Anspruch 1, dadurch gekennzeichnet, daß die Mittel zur Erzeugung eines Vibrationsfelds aus einem Vibriertisch bestehen, auf dem der Behälter angeordnet ist.

## Claims

1. Device for detecting the change of viscosity of a fluid having the properties of an electrolyte,
characterized in that it comprises : an electrically insulating receptacle (1) with two electrodes of different metallic nature (5, 6) intended to come into direct contact with said fluid (7) ; means (21) for generating a vibratory field inside the receptacle, and means (11 and/or 15) for detecting the variations of the current between the electrodes as a function of time after introduction of the fluid into the receptacle (1), said variations revealing a voltage drop which is artificially restrained until a certain point (02), from which the voltage drop resumes a normal course, and showing that said change has occurred.

2. The device as claimed in claim 1,
characterized in that said detection means comprise a closed circuit connecting the electrodes (5, 6) to a resistance (10) for causing polarization thereof and means (11, 15) for measuring the current variation between the electrodes (5, 6) which is a function of the change of viscosity.

3. The device as claimed in claim 1, for measuring the coagulation time of a blood sample (7), placed in contact with an apropriate reagent (22),
characterized in that said reagent (22) is deposited in the form of a uniform layer on surfaces bathed by the electrolyte or of several superimposed layers (polyreagent).

4. The device as claimed in claims 2 and 3,
characterized in that said means (11, 15) for measuring the voltage variation comprise means for determining the time (t0) of formation of a current between the electrodes when the blood is introduced into the receptacle and the time (t1) when the current undergoes a variation which is characteristic of the coagulation.

5. Disposable assembly intended to be used as element of the apparatus as claimed in claim 3,
characterized in that it comprises a plastic material receptacle (1) and two electrodes (5, 6) adapted for cooperating removably with the means (21) for generating a vibratory field and the means (11, 15) for detecting the variation of the current between the electrodes (5, 6).

6. The apparatus as claimed in claims 2 and 3,
characterized in that it comprises a flat insulating support (32) on which are printed two electric circuits forming the conducting electrodes (30, 31), these electrodes being interdigitated so as to form therebetween an insulating space serving as receptacle for a drop of blood.

7. The apparatus as claimed in claim 6,
characterized in that at least one of the faces of the printed circuits forming the electrodes (30, 31) constitutes a support for the reagent or reagents.

8. The apparatus as claimed in claim 1,
characterized in that said means for generating a vibratory field are formed by a piezoelectric generator producing acoustic vibrations having a frequency of a few hundred Hertz.

9. The apparatus as claimed in claim 1,
characterized in that the means for generating a vibratory field are formed by a vibrating table on which the receptacle is placed.
